# EUROPEAN PATENT APPLICATION

(11) **EP 2 559 425 A1**
(43) Date of publication of application: **20.02.2013**
(21) Application number: 11177837.9
(22) Date of filing: 17.08.2011
(51) Int. Cl.: A61K 8/46, A61Q 9/04

(54) **Depilatory method and kit**

(71) Applicant: The Procter & Gamble Company, Cincinnati, OH 45202 (US)
(72) Inventor: Smith, Paul, James, Whitton, Middlesex TW2 6EB (GB); Trani, Marina, Richmond, Surrey TW9 4BP (GB); Jakubovic, David, Andrew, Staines, Middlesex TW18 2DE (GB); Dring, Neil, Charles, Medmenham, Buckinghamshire SL7 2EZ (GB)
(74) Representative: Wilding, Richard Alan

(57) **Abstract**

A depilatory kit is provided comprising:
(a) a depilatory composition comprising an effective amount of a keratin reducing agent;
(b) a sheet of occlusive material which is separate from the depilatory composition.

## Description

### FIELD OF THE INVENTION

The present invention relates to a depilatory method and kit.

### BACKGROUND OF THE INVENTION

Depilatory compositions are cosmetic hair removal formulations. They are generally aqueous compositions comprising keratin reducing agents, which reducing agents attack the disulphide bonds in hair to weaken it, such that subsequent gentle scraping and/or wiping completes severance of the hair from the skin and effects hair removal. Commercially, the most common keratin reducing agents are thioglycolates.

An unwanted side effect of chemical depilation is that, in addition to contacting hair to be removed, the depilatory composition comes into contact with skin. This skin contact combined with the alkaline conditions needed for effective hair removal may give rise to skin irritation.

US 2004/0219118 discusses the problem of skin irritation and proposes treatment with a "lipophilic" material before application of a thioglycolate-based reactive depilatory composition. The lipophilic materials exemplified in this patent application are oils, such as mineral oil.

### SUMMARY OF THE INVENTION

According to a first aspect of the invention, a depilatory kit is provided, comprising:
(a) a depilatory composition comprising an effective amount of a keratin reducing agent;
(b) a sheet of occlusive material which is separate from the depilatory composition.

According to a second aspect of the invention, a method of removing hair from skin is provided, comprising the steps of:
(a) applying a depilatory composition to an area of skin on which unwanted hair is growing, the depilatory composition comprising a keratin reducing agent;
(b) subsequently applying a sheet of occlusive material to cover the depilatory composition.

### DETAILED DESCRIPTION OF THE INVENTION

Applicants have established that the subsequent addition of a sheet of occlusive material to cover the previously applied depilatory composition prevents water loss from the depilatory composition while the depilatory composition is in contact with the keratinous tissue and thus prevents the depilatory composition from drying out. Water loss from the depilatory composition lowers the water concentration, thereby increasing the concentration of active ingredients and bases present. Increasing the concentration of active ingredients and bases present may result in increased irritation to the skin.

According to the invention, the sheet of occlusive material may comprise a polymer, a metal foil, paper, woven or non-woven cloth or a mixture of these materials. The sheet may comprise a single layer or comprise a laminate of two or more layers.

Preferably, the sheet of occlusive material comprises a polymer. Advantageously, the polymer may comprise a polypropylene (PP), polyethylene (PE, including high density PE (HDPE) and Linear Low Density PE (LLDPE)), polyethylene terephthalate (PET), polyvinylchloride (PVC), polyamide (PA), polycarbonate, polyurethane, cellulose acetate, polychloropene, polysulfone, polytetrafluoroethylene (PTFE), polyvinyl acetate (PVA), polystyrene, polyphenylene oxide (PPO), acrylonitrile butadiene styrene (ABS), acrylic; acrylonitrile styrene acrylate (ASA), ethylene vinyl alcohol (EVA), natural rubber, latex, nylon, nitrile, silicone, thermo plastic elastomers (TPE) and mixtures thereof. Preferably the sheet of occlusive material comprises a polyolefin, more preferably a polyethylene or polypropylene and even more preferably high density polyethylene.

Advantageously, the sheet of occlusive material possesses a rigidity in the range of from 0.05 g/cm to 5.00 g/cm, preferably from 0.05 g/cm to 3.00 g/cm, more preferably from 0.08 g/cm to 1.80 g/cm, even more preferably from 0.10 g/cm to 0.80 g/cm and more preferably still from 0.20 g/cm to 0.60 g/cm. A rigidity within these ranges bestows desirable handling and conformability attributes of the sheet of occlusive material. In particular, a rigidity within this range may prevent the sheet of occlusive material from collapsing under gravity or folding, while still allowing it to conform to the surface to which it is applied without folding or crinkling.

Rigidity can be measured using the American Standard Test Method (ASTM) D2923-06, Method B (i.e. using a powder to reduce the effect of static electricity) on a Handle-O-Meter, model #211-300, available from Thwing-Albert Instrument Co. of Philadelphia, Pa, USA. The rigidity is expressed as grams per centimetre of sample width. Samples of the sheet of occlusive material were prepared as 10.16 cm (4 inch) by 10.16 cm (4 inch) test specimens with edges parallel to the machine direction and transverse direction for substrates with directionality. Three rigidity measurements were determined on the same side of fresh test specimens orientated in the same substrate direction. A further three rigidity measurements were taken on the same side of fresh test specimens oriented at 90° to the first orientation. These six measurements were repeated on the opposite side to the first six measurements, on fresh test samples. The 12 rigidity measurements were then averaged and reported to 0.01 g/cm.

The rigidity of a sheet of occlusive material is a function of sheet thickness and inherent modulus of elasticity. Different materials have different moduli of elasticity. Based upon the material or materials that the sheet of occlusive material comprises, a thickness should be selected that enables the desired rigidity of the substrate to be achieved. The sheet of occlusive material preferably has a thickness from 80 µm to 12 µm, more preferably from 50 µm to 15 µm, even more preferably from 40 µm to 16 µm, and more preferably still from 30 µm to 17 µm.

The sheet of occlusive material may be a laminate comprising at least two materials, including non-wovens, paper, board, metal based substrates (eg aluminium foil); flocking or topical coatings (e.g. surfactants, printing), closed or open cell foams or substrates described herein above. At least one of the materials is an occlusive material.

The sheet of occlusive material may be manufactured by any suitable method, including casting, injection moulding, co-injection moulding, over moulding, in-mold assembly, compression moulding, blow moulding, casting thermo or vacuum forming and where appropriate may be laminated by heat welding (which may further include the use of pressure, ultrasonic forces and radio or high frequencies), co-extrusion; adhesives, electro static adhesions (such as flocking by fibres) and topical surface applications.

The sheet of occlusive material may comprise indicia or markings to aid the placement by the user. The indicia or markings may include showing the user a suitable site at which to hold the sheet of occlusive material, to enable alignment of the article with the depilatory composition on the surface of the body, to provide safety information or guidance on applying or removing the sheet of occlusive material or the application time of the depilatory composition and sheet of occlusive material. Non-limiting examples of indicia or markings include a mark to enable alignment of the sheet of occlusive material with the corner of a mouth or the centre of the armpit, or designs upon a region of the sheet of occlusive material that should overlap the perimeter of the depilatory composition upon placement.

At least a portion of the sheet of occlusive material may be transparent, alternatively the entirety of the sheet of occlusive material may be transparent and alternatively again the sheet of occlusive material may have at least one region (which may be a partial or complete border region around the edge of the sheet of occlusive material) that is not transparent and at least one second region that is transparent. As used herein, the term "transparent" refers to materials, articles and/or compositions through which it is possible to see with the naked eye . Applicants have found that selecting a sheet of occlusive material with at least a portion that is transparent enables the user to more accurately position the sheet of occlusive material over the depilatory composition on the surface of the body. Additionally, unwanted hairs selected for treatment become pressed against the sheet of occlusive material by the application of the sheet of occlusive material on the depilatory composition, enabling the user to see them and consequently to visually assess progress being made by the depilatory activity rather than relying on estimated timings alone, as the hairs curl and crinkle during exposure to the depilatory composition. This aids the user in improving depilatory efficacy while reducing the risk of skin irritation. A sheet of occlusive material in which the entirety of the sheet is transparent enables the user to see the entire area of the depilatory composition that has been applied to the body to enable accurate positioning, while having regions of transparent and non-transparent of the sheet of occlusive material may provide indications of suitable sites for holding the article, guides to aid placement of the article, or supports that increase the rigidity of the depilatory article to aid handling. It may be advantageous for both the sheet of occlusive material and the depilatory composition to have at least one region that is transparent and more preferably still to have the same region of the sheet of occlusive material and the depilatory composition to be transparent.

It may also be advantageous to use a coloured sheet of occlusive material in order to enable the user to readily distinguish the areas of the depilatory composition that have been covered from those that have not been covered. Alternatively, the depilatory composition may be coloured or alternatively again, both the sheet of occlusive material and the depilatory composition may be coloured. Preferably, the sheet of occlusive material is at least partially coloured such that the depilatory composition and unwanted hair is clearly visible. Typical hair colours are white, grey, black, brown, red (ginger) and blonde. Accordingly, in a preferred embodiment, the substrate of the depilatory article is at least partially coloured and may be coloured throughout, more preferably in a colour not matching a typical hair colour or a colour complementary to typical hair colours. Advantageously, the substrate is colourless, coloured green, blue, purple (violet) or pink, more preferably the substrate is colourless, coloured green or blue and even more preferably the substrate is colourless or coloured blue.

The sheet of occlusive material may also comprise a fragrance or an odor masking agent to reduce the malodor that arises from the typically sulphur-containing keratin reducing agents that provide activity to the depilatory composition. The fragrance or odor masking agent may be included in the sheet of the occlusive material by any suitable means, including topically coating of the fragrance, addition of fragrance or ordor masking agent substrates (such as fragranced stickers) or inclusion within the sheet of occlusive material (for example, within a master batch).

The sheet of occlusive material may be cut to standard shapes and sizes for a specific body area during the manufacturing process or by the user to tailor the size and shape of the sheet of occlusive material to the area the user requires to removal unwanted hair.

Any depilatory composition comprising a suitable keratin reducing agent may be used in the present method and included in the present kit. Non-limiting examples of suitable keratin reducing agents include: sulphide salts such as Li₂S, Na₂S, K₂S, MgS, CaS, SrS or BaS, hydrogen sulphide salts such as NaSH or KSH; thioglycol; thioglycerol; thioglycolamide; thioglycolhydrazide; thioglycolic acid; thioglycolate salts (such as potassium thioglycolate, calcium thioglycolate, ammonium thioglycolate, diammonium dithioglycolate, glyceryl monothioglycolate, or monoethanolamine thioglycolate); thiosalicylic acid; thiomalic acid; ammonium thiolactate; monoethanolamine thiolactate; dithioerythritol; 2-mercaptopropionic acid; 1,3-dithiopropanol; glutathione; dithiothreitol; cysteine; homocysteine; N-acetyl-L-cysteine and cysteamine. Advantageously, the keratin reducing agent is comprised within the depilatory composition in an amount from 0.3% to 20%, preferably from 0.8% to 15%, more preferably from 1% to 10% by weight of the depilatory composition.

Advantageously, the depilatory composition may comprise at least one thioglycolate salt or thioglycollic acid acting as a hair removal agent when the depilatory composition is applied to unwanted hair. Preferably, the depilatory composition comprises sodium, potassium, magnesium, calcium, beryllium, strontium, zinc, monoethanolamine, ammonium, tetralkylammonium, imidazolium, pyridinium, phosphonium or glyceryl thioglycolate salts, or mixtures thereof, which may include dianion forms of thioglycolate. More preferably, the depilatory composition comprises at least one of sodium, potassium, magnesium or calcium thioglycolate, or mixtures thereof. Even more preferably the depilatory composition comprises potassium or calcium thioglycolate, or mixtures thereof.

The pH of the depilatory composition may advantageously be in the range of from 6 to 13.8, preferably from greater than 7 to 13, more preferably from 9 to 12.9, even more preferably from 10 to 12.8, even more preferably still from 12 to 12.75 and yet more preferably from 12.3 to 12.6 to improve the efficacy of the active ingredient. The depilatory composition may, in a preferred embodiment, comprise at least one base to control the pH. Preferably, the depilatory composition comprises potassium hydroxide; sodium hydroxide; lithium hydroxide; calcium hydroxide; barium hydroxide; caesium hydroxide; sodium hydroxide; ammonium hydroxide; strontium hydroxide; rubidium hydroxide; magnesium hydroxide; zinc hydroxide; sodium carbonate; pyridine; ammonia; alkanolamides (including monoethanolamine, diethanolamine, triethanolamine), phosphates (including tetrasodium phosphate), arginine or mixtures thereof. More preferably, the depilatory composition comprises at least one buffering base, even more preferably the depilatory composition comprises calcium hydroxide, magnesium hydroxide; barium hydroxide; strontium hydroxide; zinc hydroxide; arginine or mixtures thereof. Still more preferably the depilatory composition comprises calcium hydroxide; magnesium hydroxide, zinc hydroxide, sodium hydroxide, potassium hydroxide or mixtures thereof. Even more preferably still, the depilatory composition comprises calcium hydroxide, sodium hydroxide or mixtures thereof.

In an advantageous embodiment, the base is present at a concentration of from 0.1% to 10.0%, more preferably from 0.5% to 8.0% and even more preferably from 1.0% to 5.0%, by weight of the depilatory composition.

The concentration of water in the depilatory composition is preferably at least 40%, more preferably from 50% to 98%, even more preferably from 60% to 95% and even more preferably still from 70% to 90%, by weight of the depilatory composition.

The aqueous depilatory composition may comprise at least one monovalent cation, preferably a monovalent metal cation. The monovalent cations such as those derived from monovalent cation containing salts are able to displace the cation of the thioglycolate salt and further enhance dissociation of said thioglycolate salt. This increases the amount of deprotonated thioglycolate formed from the thioglycolate salt and therefore increases the effectiveness of the aqueous depilatory composition. Sources of monovalent cations include potassium, sodium, lithium, ammonium, tetraalkyl ammonium and imidazolium salts, which may be a component of another ingredient, for example a thickening system or skin care active. Preferred sources of monovalent cations include potassium and sodium salts.

The quantity of monovalent cations (or in a preferred embodiment above monovalent metal cations) per unit area of the aforementioned coated region is from 6 × 10⁻⁷ mol/cm² to 6 × 10⁻⁶ mol/cm², preferably from 1.5 × 10⁻⁶ mol/cm² to 4.5 × 10⁻⁶ mol/cm² and more preferably from 2.25 × 10⁻⁶ mol/cm² to 3.75 × 10⁻⁶ mol/cm². Without wishing to be bound by theory, the applicants believe that within this narrow range of monovalent cation dosage, the efficacy of the depilatory composition may be increased while remaining within the bounds of tolerance of human skin, consequently reducing irritation. The selection of keratin reducing agent and optional ingredients including the base may be made considering the quantity of monovalent cations or monovalent metal cations achieved.

The depilatory composition may optionally comprise a thickening agent. A representative but not exhaustive list can be found in "The Encyclopaedia of Polymers and Thickeners for Cosmetics" compiled and edited by Robert Y. Lochhead, PhD and William R. Fron, Department of Polymer Science, University of Southern Mississippi. Exemplary classes of thickening agents include gums, carbomers, polymers and copolymers of acrylic acid, associated thickeners, layered silicates/clays and natural polymers (including polysaccharides). One or more thickening agents may be included in the aqueous depilatory composition. The thickening agent may be present at a level of from 0.01% to 20%, preferably from 0.1% to 10% by weight of the depilatory composition.

Advantageously, the depilatory composition may have a yield point from 10 Pa to 2000 Pa, more preferably from 30 Pa to 1200 Pa, even more preferably from 45 Pa to 500 Pa and even more preferably still from 60 Pa to 300 Pa, when measured via a stress controlled amplitude sweep at a frequency of 1 Hz and a temperature of 25°C. The yield point described is defined as the 5% decrease in magnitude of the elastic modulus G' linear viscoelastic plateau value as measured on a TA1000 Rheometer, available from TA Instruments of New Castle, Delaware, USA. The rheological properties of the depilatory composition may be altered by changing the concentration or identity of the thickening system and the water content of the depilatory composition.

The depilatory composition may also include other skin care ingredients such as conditioning agents selected from the group consisting of humectants, moisturizers, or skin conditioners (including mineral oil; almond oil; chamomile oil; jojoba oil; avocado oil; shea butter, niacinamide and glycerine); skin rejuvenation compositions (for example targeted for fine lines, wrinkles and uneven skin tone, including retinoids), cosmetic compositions; anti-inflammatory agents (including corticosteroids); anti-oxidants (including flavonoids) radical scavengers; sunscreen agents; skin cooling or warming agents and the like. The depilatory composition may comprise one or more skin care ingredients present in an amount of from 0.001% to 10%, more preferably from 0.01% to 7%, and even more preferably from 0.025% to 5%, by weight of the depilatory composition.

An accelerant may be employed in the depilatory composition, which accelerates the rate of depilatory action of the depilatory agent. Suitable accelerants include, but are not limited to, urea; thiourea; dimethyl isosorbide; arginine salts; ethoxydiglycol; propylene glycol and methylpropyldiol. The accelerant may be present in a concentration range of from 0.5% to 10%, more preferably from 2% to 8% and even more preferably from 2% to 5% by weight of the depilatory composition.

The depilatory composition may further comprise components known, conventionally used, or otherwise effective for use in cosmetic compositions, such as dyes; pigments (including ultra marines and talc); anionic, cationic, non-ionic and/or amphoteric or zwitterionic surfactants, polymers (including hydrophobically modified polymers); dispersing agents; solvents; lubricants; fragrances; preservatives; chelants, proteins and derivatives thereof, plant materials (e.g. aloe, chamomile and henna extracts); silicones (volatile or non-volatile, modified or non-modified); film-forming agents; film forming promoters and mixtures thereof.

The depilatory composition may be formulated in any common delivery form, such as a cream or lotion.

In addition to the depilatory composition and the occlusive material, the kit according to the second aspect of the invention may comprise one or more of:
(a) A removal composition and/or a removal wipe;
(b) Means for removal of the depilatory composition and the sheet of occlusive material following use, which means may comprise a tool, such as a spatula;
(c) A post-treatment composition skin care composition to be applied to the area of skin from which hair has been removed. Such a post-treatment skin care composition may comprise ingredients to promote skin conditioning; moisturizers, skin rejuvenation compositions (targeted for fine lines, wrinkles and uneven skin tone, for example), cosmetic compositions (e.g., foundation, rouge), sunscreens and the like. The post-treatment skin care composition may be leave-on or a rinse-off composition.
(d) Instructions regarding how to use the various elements of the kit, which instructions may comprise one or more elements of the method as defined herein.

Prior to applying the method or using the kit according to the present invention, a user should advantageously remove all make-up from the skin, to ensure good adherence and effective application of both the depilatory composition and the sheet of occlusive material.

Preferably, the depilatory composition is applied to the skin in an amount per unit area from 0.001 g/cm² to 0.6 g/cm², preferably from 0.003 g/cm² to 0.5 g/cm², more preferably from 0.005 g/cm² to 0.3 g/cm² and more preferably still from 0.2 g/cm² to 0.01 g/cm². The depilatory composition may be applied to the skin by any means, including the user's finger or hand, from the depilatory composition container such as a tube, bottle or sachet, via a tool (including spatulas, pipettes, brushes, sponges, tines, foams, non-wovens, wovens sintered elements and pads) or dispensed via an applicator (including rollers, sprays / atomizers, pistons, pumps, pressurized containers, tilt valves and integrated devices). Preferably, the depilatory composition is applied to the skin via a tool or dispensed via an applicator and even more preferably from an applicator.

Subsequent to, but as soon as possible after application of the depilatory composition, the sheet of occlusive material is applied by the user. The sheet of occlusive material should be applied such that the surface of the depilatory composition is fully covered and may extend beyond the perimeter of the depilatory composition by up to 8 mm, preferably by up to 5 mm and more preferably by up to 2 mm at every point on the perimeter. The sheet of occlusive material is carefully placed over the area to which depilatory cream has been applied either by hand or via a dispensing applicator (such as a roller).

Following application, the depilatory composition and the sheet of occlusive material are advantageously left in place for at least 1 minute, preferably from 1 to 10 minutes, more preferably from 3 to 10 minutes, depending on the thickness of the hair and the hair removal efficacy of the depilatory composition (which, in turn, is dependent upon the concentration of keratin reducing agent in the depilatory composition).

Once the depilatory composition has been in place for sufficient time to be effective, then it and the sheet of occlusive material are advantageously removed. The sheet of occlusive material may advantageously be peeled off, after which the depilatory composition may be removed using one or more of a cotton wool ball, pad or wand, a tissue, a cloth, or a tool, such as a spatula or a scraper.

Advantageously, the skin from which hair has been removed is then rinsed with water.

In an advantageous subsequent step, a post-treatment skin care composition may be applied to the area of skin from which hair has been removed. Such a post-treatment skin care composition may comprise ingredients to promote skin conditioning; moisturizers, skin rejuvenation compositions (targeted for fine lines, wrinkles and uneven skin tone, for example), cosmetic compositions (e.g., foundation, rouge), sunscreens and the like. The post-treatment skin care composition may be leave-on or a rinse-off composition.

### Examples

Depilatory composition example:

| Depilatory Ingredient | % w/w |
|---|---|
| DI water | 84.42 |
| Acrylic acid / VP crosspolymer (Ultrathix P-100)¹ | 3.00 |
| Calcium hydroxide² | 4.50 |
| Calcium thioglycolate trihydrate³ | 6.00 |

| | |
|---|---|
| 1 Ultrathix P-100 available from International Specialty Products Inc. (ISP) 2 Calcium hydroxide Reag. Ph. Eur. puriss. p.a. available from Sigma-Aldrich Co. 3 Calcium thioglycolate trihydrate 99.8% available from BRUNO BOCK Chemische Fabrik GmbH & Co. | |

### Examples of sheets of occlusive material

| **Substrate Material** | **Thickness [microns]** | **Rigidity [g/cm]** |
|---|---|---|
| HDPE | 13 | 0.13 |
| HDPE | 18 | 0.33 |
| HDPE | 36 | 1.05 |
| LLDPE | 23 | 0.23 |
| PP | 18 | 0.46 |

| | | |
|---|---|---|
| [HDPE is a mixture of LBI 85% M6030 and Exxon Mobil 15% LD2001 manufactured on a Merritt-Davis casting line] [LLDPE is Exxon Mobil 15% LD2001 manufactured on a Merritt-Davis casting line] [PP is Basell PH835 manufactured on a Merritt-Davis casting line] | | |

A sheet of occlusive material (36 microns HDPE, 1.55cm in width and 7.5cm in length) was applied onto a depilatory composition such that the sheet of occlusive material extended over the depilatory composition by 2mm at every point on the perimeter to prevent water from being lost from the depilatory composition during the application period.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A depilatory kit comprising:
(a) a depilatory composition comprising an effective amount of a keratin reducing agent;
(b) a sheet of occlusive material which is separate from the depilatory composition.

2. The depilatory kit of claim 1, wherein the sheet of occlusive material comprises polymer, metal foil, paper, woven material, non-woven material, or mixtures thereof.

3. The depilatory kit of claim 1 or 2, wherein the sheet of occlusive material comprises a polymer.

4. The depilatory kit of claim 3, wherein the polymer comprises a polyolefin, preferably a polyethylene or polypropylene and more preferably high density polyethylene.

5. The depilatory kit of any preceding claim, wherein the sheet of occlusive material possesses a rigidity in the range of from 0.05 g/cm to 5.00 g/cm, preferably from 0.05 g/cm to 3.00 g/cm, more preferably from 0.08 g/cm to 1.80 g/cm, even more preferably from 0.10 g/cm to 0.80 g/cm and more preferably still from 0.20 g/cm to 0.60 g/cm.

6. The depilatory kit of any preceding claim, wherein the sheet of occlusive material has a thickness from 80 nm to 12 µm, preferably from 50 nm to 15 µm, more preferably from 40 nm to 16 µm, and more preferably still from 30 nm to 17 nm.

7. The depilatory kit of any preceding claim, wherein at least a portion and preferably the entirety of the sheet of occlusive material is transparent.

8. The depilatory kit of any preceding claim, wherein at least a portion and preferably the entirety of the sheet of occlusive material is coloured.

9. The depilatory kit of any preceding claim, wherein the sheet of occlusive material comprises indicia or markings to guide the user in holding, positioning, applying, using or removing the sheet of occlusive material.

10. The depilatory kit of any preceding claim, wherein the keratin reducing agent comprises a thioglycolate salt, preferably potassium or calcium thioglycolate, or mixtures thereof.

11. The depilatory kit of any preceding claim, additionally comprising the following instructions:
(a) An instruction to apply the depilatory composition to an area of skin to be depilated;
(b) An instruction to apply the sheet of occlusive material over the pre-applied depilatory composition to cover the depilatory composition.
(c) Optionally, an instruction to leave the depilatory composition and the sheet of occlusive material in place for a period of at least 1 minute, preferably from 1 to 10 minutes, more preferably from 3 to 10 minutes;
(d) Optionally, an instruction to remove the depilatory composition and the sheet of occlusive material;
(e) Optionally an instruction to treat the area of skin which has been depilated with a post-treatment skin care composition.

12. A method of removing hair from skin, preferably facial skin, comprising the steps of:
(a) applying a depilatory composition to an area of skin on which unwanted hair is growing, the depilatory composition comprising a keratin reducing agent;
(b) subsequently applying a sheet of occlusive material as defined in any of claims 2 to 9 to cover the depilatory composition.

13. The method of any claim 12, wherein the quantity of depilatory composition applied per unit area of skin is from 0.001 g/cm² to 0.6 g/cm², preferably from 0.003 g/cm² to 0.5 g/cm², more preferably from 0.005 g/cm² to 0.3 g/cm² and more preferably still from 0.2 g/cm² to 0.01 g/cm²_{.}

14. The method of claim 10 or 11, wherein the sheet of occlusive material is applied to completely cover area of skin ro which the depilatory composition has been applied.

15. The method of any of claims 10 to 14, whereby the sheet of occlusive material extends beyond the perimeter of the depilatory composition by up to 8mm, preferably up to 5mm and more preferably up to 2mm at every point on the perimeter.
